(19) Europäisches Patentamt
European Patent Office
Office européen des brevets

(11) EP 3 644 735 B1

(12) # FASCICULE DE BREVET EUROPEEN

(45) Date de publication et mention de la délivrance du brevet:
**07.04.2021 Bulletin 2021/14**

(21) Numéro de dépôt: **18732806.7**

(22) Date de dépôt: **27.06.2018**

(51) Int Cl.:
*A01N 35/02* (2006.01) *A01P 3/00* (2006.01)
*A01N 37/52* (2006.01) *A01N 43/40* (2006.01)
*A01N 47/36* (2006.01) *A01N 59/16* (2006.01)

(86) Numéro de dépôt international:
**PCT/EP2018/067302**

(87) Numéro de publication internationale:
**WO 2019/002394 (03.01.2019 Gazette 2019/01)**

(54) **MELANGE ANTIMICROBIEN CONTENANT DU 4-(3-ETHOXY-4-HYDROXYPHENYL)BUTAN-2-ONE ET UN SECOND AGENT ANTIMICROBIEN, ET COMPOSITION COSMETIQUE LE CONTENANT**

ANTIMIKROBIELLES GEMISCH ENTHALTEND 4-(3-ETHOXY-4-HYDROXYPHENYL)BUTAN-2-ON UND EINEN ZWEITEN ANTIMIKROBIELLEN WIRKSTOFF, SOWIE KOSMETISCHE ZUSAMMENSETZUNGEN, DIE DIESES ENTHÄLT

ANTIMICROBIAL MIXTURE CONTAINING 4-(3-ETHOXY-4-HYDROXYPHENYL)BUTAN-2-ONE AND A SECOND ANTIMICROBIAL AGENT, AND COSMETIC COMPOSITIONS CONTIANING IT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.06.2017 FR 1756182**
**18.08.2017 FR 1757747**
**18.08.2017 FR 1757748**
**18.08.2017 FR 1757749**
**13.09.2017 FR 1758493**

(43) Date de publication de la demande:
**06.05.2020 Bulletin 2020/19**

(73) Titulaire: **L'Oreal**
**75008 Paris (FR)**

(72) Inventeurs:
- **MENARD-SZCZEBARA, Florence**
  **94152 Chevilly-Larue (FR)**
- **CUPFERMAN, Sylvie**
  **94152 Chevilly-Larue (FR)**
- **MALET, Gael**
  **94152 Chevilly-Larue (FR)**
- **GALVAN, Julien**
  **94152 Chevilly-Larue (FR)**

(74) Mandataire: **Rivière, François Armand**
**L'Oréal**
**Service DIPI**
**9 Rue Pierre Dreyfus**
**92110 Clichy (FR)**

(56) Documents cités:
**WO-A1-2011/039445**
**US-A1- 2009 306 154**
**US-A1- 2010 119 461**

EP 3 644 735 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention a pour objet un mélange antimicrobien contenant du 4-(3-éthoxy-4-hydroxyphényl)butan-2-one et d'un composé additionnel particulier, ainsi qu'une composition cosmétique contenant un tel mélange.

Le 4-(3-éthoxy-4-hydroxyphényl)butan-2-one (composé cétonique) est une substance intéressante en tant que conservateur de compositions cosmétiques pour protéger les compositions de la contamination microbienne, comme décrit dans la demande WO2011/039445.

Toutefois, il est souhaitable de pouvoir incorporer ledit composé cétonique en concentration réduite dans des compositions, notamment cosmétiques ou dermatologiques, tout en conservant une bonne performance de conservation antimicrobienne. Il est donc recherché à cet effet des associations du composé cétonique avec d'autres composés présentant une efficacité antimicrobienne.

Les inventeurs ont découvert, de façon inattendue, que l'association du 4-(3-éthoxy-4-hydroxyphényl)butan-2-one avec un composé additionnel choisi parmi un sel de métal monovalent ou divalent de la pyrithione, la piroctone olamine, le diazolidinylurée, un composé hexamidine, l'imidazolidinylurée, permet d'obtenir un mélange antimicrobien présentant une synergie d'activité antimicrobienne, en particulier sur au moins sur les levures, en particulier sur *Candida albicans.*

Les résultats des exemples décrits ci-après montrent l'activité antimicrobienne synergique obtenue avec les mesures de concentrations minimales inhibitrices (CMI) faites avec plusieurs mélanges. L'activité antimicrobienne est considérée comme étant synergique lorsque le mélange antimicrobien permet d'obtenir un pourcentage de croissance de la souche inférieur ou égal à 20 %, voire inférieur à 25 %.

L'association du 4-(3-éthoxy-4- hydroxyphényl)butan-2-one avec un sel de métal monovalent ou divalent de la pyrithione, en particulier ledit sel de zinc, permet d'obtenir un mélange antimicrobien présentant une synergie d'activité antimicrobienne, en particulier sur bactéries Gram +, notamment sur *Staphylococcus aureus,* et sur les levures, en particulier sur *Candida albicans.*

L'association du 4-(3-éthoxy-4-hydroxyphényl)butan-2-one avec la piroctone olamine permet d'obtenir un mélange antimicrobien présentant une synergie d'activité antimicrobienne, en particulier sur les levures, notamment sur *Candida albicans.*

La demande FR-A-2962333 décrit une composition cosmétique pour le traitement de la peau grasse comprenant un composé 2-alcoxy-4-alkylcétonephénol et une huile essentielle. La composition peut comprendre un actif additionnel pour le soin des peaux grasses, tels que des agents antimicrobiens parmi lesquels est mentionné la piroctone olamine. Ce document ne décrit pas spécifiquement un mélange antimicrobien constitué de l'association du 4-(3-éthoxy-4-hydroxyphényl)butan-2-one avec la piroctone olamine, et ne suggère pas non plus qu'un tel mélange présente une activité antimicrobienne synergique sur les levures, notamment sur *Candida albicans.*

L'association du 4-(3-éthoxy-4- hydroxyphényl)butan-2-one avec du diazolidinylurée permet d'obtenir un mélange antimicrobien présentant une synergie d'activité antimicrobienne sur les levures, en particulier sur *Candida albicans.*

L'association du 4-(3-éthoxy-4- hydroxyphényl)butan-2-one avec d'un composé hexamidine permet d'obtenir un mélange antimicrobien présentant une synergie d'activité antimicrobienne sur les levures, en particulier sur *Candida albicans.*

La demande FR-A-2962333 décrit une composition cosmétique pour le traitement de la peau grasse comprenant un composé 2-alcoxy-4-alkylcétonephénol et une huile essentielle. La composition peut comprendre un actif additionnel pour le soin des peaux grasses, tels que des agents antimicrobiens parmi lesquels est mentionné l'hexamidine iséthionate. Ce document ne décrit pas spécifiquement un mélange antimicrobien constitué de l'association du 4-(3-éthoxy-4-hydroxyphényl)butan-2-one avec l'hexamidine iséthionate, et ne suggère pas non plus qu'un tel mélange présente une activité antimicrobienne synergique sur les levures, notamment sur *Candida albicans.*

L'association du 4-(3-éthoxy-4- hydroxyphényl)butan-2-one avec de l'imidazolidinylurée permet d'obtenir un mélange antimicrobien présentant une synergie d'activité antimicrobienne, en particulier sur les moisissures, notamment sur *Aspergillus niger, et* sur les levures, en particulier sur *Candida albicans.*

De façon plus précise, l'invention a pour objet un mélange antimicrobien comprenant, ou constitué (ou consistant en), de la 4-(3-éthoxy-4-hydroxyphényl)butan-2-one et d'un composé additionnel choisi parmi un sel de métal monovalent ou divalent de la pyrithione, la piroctone olamine, le diazolidinylurée, un composé hexamidine choisi parmi le diiséthionate d'hexamidine, l'hexamidine diparabène et l'hexamidine parabène, l'imidazolidinylurée.

L'invention a également pour objet une composition, notamment cosmétique ou dermatologique, comprenant dans un milieu physiologiquement acceptable, ledit mélange décrit précédemment.

L'invention a encore pour objet un procédé de traitement cosmétique non thérapeutique des matières kératiniques comprenant l'application sur les matières kératiniques d'une composition telle que décrite précédemment. Le procédé peut être un procédé cosmétique de soin ou de maquillage ou de nettoyage des matières kératiniques.

L'invention a également pour objet un procédé de conservation d'une composition comprenant un milieu physiologiquement acceptable, en particulier une composition cosmétique ou dermatologique, caractérisé en ce qu'il consiste à incorporer à ladite composition un mélange antimicrobien tel que décrit précédemment.

L'invention a aussi pour objet l'utilisation du mélange antimicrobien décrit précédemment pour la conservation d'une composition comprenant un milieu physiologiquement acceptable.

La 4-(3-éthoxy-4-hydroxyphényl)butan-2-one est un composé de formule :

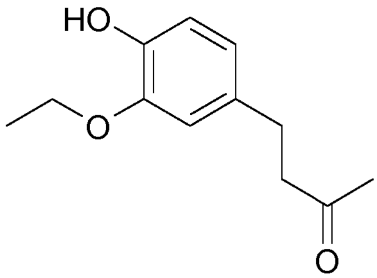

Selon un premier mode de réalisation, l'invention a pour objet un mélange antimicrobien comprenant, ou constitué (ou consistant en), de la 4-(3-éthoxy-4-hydroxyphényl)butan-2-one et d'un sel de métal monovalent ou divalent de la pyrithione.

La pyrithione est le composé 1-Hydroxy-2(1H)-pyridinethione ou 2-pyridinethiol-1-oxyde.

Les sels de pyrithione utilisés dans le cadre de l'invention sont les sels de métal monovalent et de métal divalent (appelés également sel de pyrithione dans la suite de la description) tels que les sels de sodium, calcium, de magnésium, de baryum, de strontium, de zinc, de cadmium, d'étain et de zirconium. Les sels préférés sont les sels de sodium ou de zinc, et préférentiellement le sel de zinc (zinc pyrithione).

Le zinc pyrithione correspond au composé de formule chimique ci-dessus (n° CAS : 13463-41-7) :

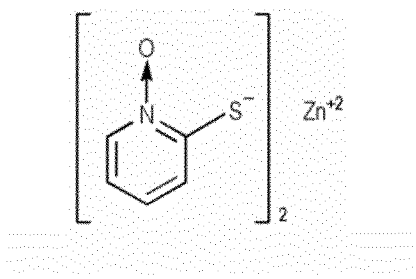

Avantageusement, la 4-(3-éthoxy-4-hydroxyphényl)butan-2-one et le sel de métal monovalent ou divalent de la pyrithione sont présents dans ledit mélange en une teneur telle que le ratio pondéral 4-(3-éthoxy-4-hydroxyphényl)butan-2-one / sel de pyrithione va de 400 à 4500, et plus préférentiellement va de 500 à 4300.

Le mélange antimicrobien peut avoir un ratio pondéral 4-(3-éthoxy-4-hydroxyphényl) butan-2-one / sel de pyrithione allant de 400 à 2500, de préférence allant de 500 à 2200, de préférence allant de 1000 à 2200, et plus préférentiellement allant de 1800 à 2200. Un tel mélange présente une bonne activité antimicrobienne sur la bactérie Gram + *Staphylococcus aureus.*

Le mélange antimicrobien peut avoir un ratio pondéral 4-(3-éthoxy-4-hydroxyphényl) butan-2-one / sel de pyrithione allant de 800 à 4500, de préférence allant de 900 à 4300, et plus préférentiellement allant de 950 à 4200. Un tel mélange présente une bonne activité antimicrobienne sur les levures, notamment sur *Candida Albicans.*

Selon un deuxième mode de réalisation, l'invention a pour objet un mélange antimicrobien comprenant, ou constitué (ou consistant en), de la 4-(3-éthoxy-4-hydroxyphényl)butan-2-one et de la piroctone olamine.

La piroctone olamine correspond au composé au sel d'éthanolamine du 1-Hydroxy-4-methyl-6-(2,4,4-triméthylpentyl)-2(1H)-pyridone (n° CAS : 68890-66-4 ; nom INCI : PIROCTONE OLAMINE) de formule:

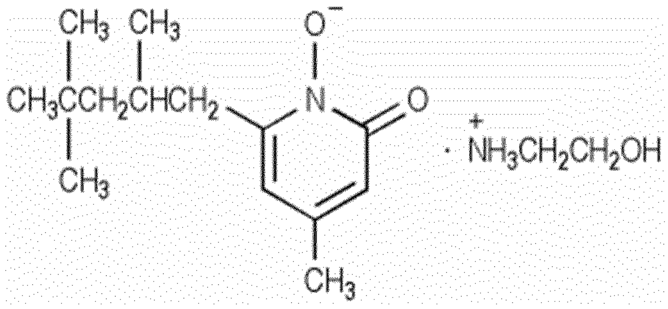

Avantageusement, la 4-(3-éthoxy-4-hydroxyphényl)butan-2-one et la piroctone olamine sont présents dans ledit mélange en une teneur telle que le ratio pondéral 4-(3-éthoxy-4-hydroxyphényl)butan-2-one / piroctone olamine va de 10 à 60, de préférence va de 15 à 50, et préférentiellement va de 18 à 45.

Selon un troisième mode de réalisation, l'invention a pour objet un mélange antimicrobien comprenant, ou constitué (ou consistant en), de la 4-(3-éthoxy-4-hydroxyphényl)butan-2-one et du diazolidinylurée.

Le diazolidinylurée correspond au composé 1-[1,3-bis(hydroxymethyl)-2,5-dioxoimidazolidin-4-yl]-1,3-bis(hydroxymethyl)urée (n° CAS : 78491-02-8), correspondant à la formule *suivante* :

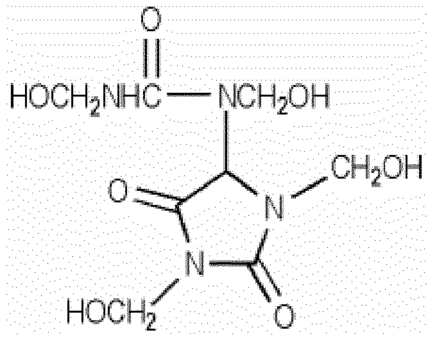

Avantageusement, la 4-(3-éthoxy-4-hydroxyphényl)butan-2-one et le diazolidinylurée sont présents dans ledit mélange en des teneurs telles que le ratio pondéral 4-(3-éthoxy-4-hydroxyphényl)butan-2-one / diazolidinylurée va de 0,5 à 2,5, de préférence va de 0,6 à 2,3, de préférence va de 0,7 à 2, et plus préférentiellement va de 0,7 à 1,8, et mieux va de 1,2 à 1,8.

Un tel mélange présente une bonne activité antimicrobienne sur les levures, notamment sur *Candida Albicans.*

Selon un quatrième mode de réalisation, l'invention a pour objet un mélange antimicrobien comprenant, ou constitué (ou consistant en), de la 4-(3-éthoxy-4-hydroxyphényl)butan-2-one et d'un composé hexamidine choisi parmi le diiséthionate d'hexamidine, l'hexamidine diparabène et l'hexamidine parabène.

De préférence, le composé hexamidine est le diiséthionate d'hexamidine (n° CAS : 659-40-5) qui a pour formule chimique :

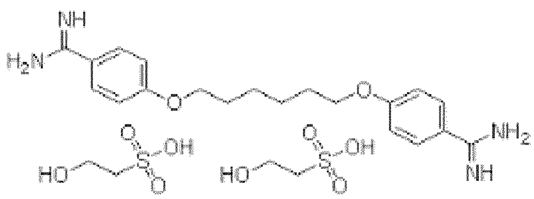

Avantageusement, la 4-(3-éthoxy-4-hydroxyphényl)butan-2-one et le composé hexamidine décrit précédemment sont présents dans ledit mélange en une teneur telle que le ratio pondéral 4-(3-éthoxy-4-hydroxyphényl)butan-2-one / composé hexamidine va de 5 à 85, de préférence va de 7 à 82, et préférentiellement va de 15 à 60.

Un tel mélange présente une bonne activité antimicrobienne sur les levures, notamment sur *Candida Albicans.*

Selon un cinquième mode de réalisation, l'invention a pour objet un mélange antimicrobien comprenant, ou constitué (ou consistant en), de la 4-(3-éthoxy-4-hydroxyphényl)butan-2-one et de l'imidazolidinylurée.

L'imidazolidinylurée correspond au composé 1,1'-méthylènebis{3-[4-(hydroxyméthyl)-2,5-dioxoimidazolidin-4-yl]urée} (n° CAS : 39236-46-9) qui a pour formule :

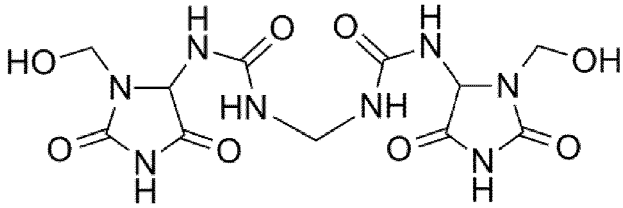

Avantageusement, la 4-(3-éthoxy-4-hydroxyphényl)butan-2-one et l'imidazolidinylurée sont présents dans ledit mélange en une teneur telle que le ratio pondéral 4-(3-éthoxy-4-hydroxyphényl)butan-2-one / imidazolidinylurée va de 0,1 à 0,8, et plus préférentiellement va de 0,15 à 0,45.

Le mélange antimicrobien peut avoir un ratio pondéral 4-(3-éthoxy-4-hydroxyphényl) butan-2-one / imidazolidinylurée allant de 0,2 à 0,8, et plus préférentiellement allant de 0,4 à 0,6. Un tel mélange présente une bonne activité antimicrobienne sur les moisissures, notamment sur *Aspergillus niger.*

Le mélange antimicrobien peut avoir un ratio pondéral 4-(3-éthoxy-4-hydroxyphényl) butan-2-one / imidazolidinylurée allant de 0,1 à 0,6, et plus préférentiellement allant de 0,15 à 0,45. Un tel mélange présente une bonne activité antimicrobienne sur les levures, notamment sur *Candida albicans.*

L'invention a aussi pour objet une composition comprenant, dans un milieu physiologiquement acceptable, le mélange antimicrobien décrit précédemment.

On entend par milieu physiologiquement acceptable, un milieu compatible avec les matières kératiniques d'êtres humains tels que la peau, le cuir chevelu, les cheveux, les ongles. Le dit milieu peut comprendre un ou plusieurs ingrédients additionnels, distinct du composé cétonique et du composé additionnel décrits précédemment.

Le composé 4-(3-éthoxy-4-hydroxyphényl)butan-2-one peut être présent dans la composition selon l'invention, en une teneur allant de 0,01 % à 5 % en poids, par rapport au poids total de la composition, de préférence allant de 0,01 % à 3 % en poids, préférentiellement allant de 0,01 à 2, 5 % en poids, et plus préférentiellement allant de 0,01 à 2 % en poids.

La composition peut comprendre au moins un ingrédient additionnel choisi parmi l'eau, les huiles, les polyols ayant de 2 à 10 atomes de carbone, les gélifiants, les tensioactifs, les polymères filmogènes, les matières colorantes, les parfums, les charges, les filtres UV, les extraits végétaux, les actifs cosmétiques et dermatologiques, et les sels.

La composition selon l'invention peut comprendre une phase aqueuse.

La composition peut comprendre de l'eau qui peut être présente en une teneur allant de 5 % à 90 % en poids, par rapport au poids total de la composition, et de préférence allant de 35% à 75% en poids.

La composition peut comprendre en outre un polyol miscible à l'eau à la température ambiante (25 °C) notamment choisi parmi les polyols ayant notamment de 2 à 10 atomes de carbones, de préférence ayant de 2 à 6 atomes de carbone, tels que la glycérine, le propylène glycol, le 1,3-propanediol, le butylène glycol, le pentylène glycol, l'hexylène glycol, le dipropylène glycol, le diéthylène glycol, la diglycérine. Avantageusement, la composition selon l'invention comprend du 1,3-propanediol, notamment en une teneur allant de 0,1 à 20 % en poids, par rapport au poids total de la composition, et de préférence allant de 0,1 à 10 % en poids, préférentiellement allant de 0,5 à 5 % en poids.

Les compositions selon l'invention peuvent se présenter sous forme d'émulsions huile dans-eau (H/E) ou eau-dans-huile (E/H) ou multiple (triple : E/H/E ou H/E/H), de solutions huileuses, de gels huileux, de solutions aqueuses, de gels aqueux, de compositions solides. Ces compositions sont préparées selon les méthodes usuelles.

Les compositions selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick ou de poudre compacte.

La composition selon l'invention peut notamment se présenter sous la forme :

- d'un produit de maquillage, notamment de la peau du visage, du corps ou des lèvres ou des cils ;
- d'un gel ou lotion après-rasage; de produit de rasage ;
- d'un déodorant (stick, roll-on, aérosol)
- d'une crème dépilatoire;
- sous la forme d'une composition d'hygiène corporelle telle qu'un gel douche ou un shampooing;
- d'une composition pharmaceutique;

- d'une composition solide telle qu'un savon ou un pain de nettoyage;
- d'une composition aérosol comprenant également un agent propulseur sous pression;
- d'une lotion de mise en plis, d'une crème ou d'un gel coiffant, d'une composition de teinture, d'une composition de permanente, d'une lotion ou d'un gel antichute, d'un après shampooing ;
- d'une composition de soin ou de nettoyage de la peau.

L'invention a aussi pour objet un procédé de préparation d'une composition, notamment cosmétique ou dermatologique, comprenant une étape de mélange du 4-(3-éthoxy-4-hydroxyphényl)butan-2-one, du composé additionnel décrit précédemment, et d'un ou plusieurs ingrédients additionnels, notamment cosmétiques ou dermatologiques, tels que ceux décrits précédemment.

L'invention est illustrée plus en détail dans l'exemple suivant. Les teneurs des ingrédients sont exprimées en pourcentage pondéral.

**Exemple 1 : Détermination de la synergie d'activité antimicrobienne en CMI**

La démonstration d'un effet de synergie d'activité antimicrobienne avec un mélange de 4-(3-éthoxy-4-hydroxyphényl)butan-2-one (appelée substance A) et de composé additionnel (appelée substance B) est réalisée par le calcul de l'indice de synergie (ou FIC index), selon la formule suivante :

$$\text{FIC Index} = (\text{CMI de A avec B} / \text{CMI de A}) + (\text{CMI de B avec A} / \text{CMI de B})$$

avec :

- CMI de A avec B : concentration minimale en produit A dans l'association A + B permettant d'obtenir un effet inhibiteur ;
- CMI de B avec A : concentration minimale en produit B dans l'association A + B permettant d'obtenir l'effet inhibiteur ;
- CMI de A : concentration minimale inhibitrice du produit A seul ;
- CMI de B : concentration minimale inhibitrice du produit B seul.

Cette formule a été décrite pour la première fois dans l'article de F.C. Kull, P.C. Eisman, H.D. Sylwestrowka, and R.L. Mayer, Applied Microbiology 9:538-541, 1961.

Pour chaque composé testé seul, la CMI est considérée comme la première concentration permettant l'obtention d'un pourcentage de croissance microbienne inférieur ou égal à 20%, voire inférieur à 25 %.

Concernant les associations testées, CMI de A avec B et CMI de B avec A sont les concentrations respectives de A et de B dans les associations permettant l'obtention d'un pourcentage de croissance microbienne inférieur ou égal à 20%, voire inférieur à 25 %.

**Interprétation du FIC Index** :

Lorsque la valeur du FIC index est inférieure ou égale à 1, on considère que l'association des composés testés présente un effet synergique.

La synthèse des résultats obtenus est présentée dans les tableaux suivants.

L'association des composés A et B a été testée sur les souches suivantes ou une partie de ces souches : *Candida albicans, Staphylococcus aureus, Aspergillus niger.*

On a utilisé la souche microbienne *Staphylococcus aureus* ATCC 6538 et un milieu de culture liquide bouillon nutritif à double concentration.

On a utilisé la souche microbienne *Candida albicans* ATCC 10231 et un milieu de culture liquide bouillon Sabouraud, à double concentration.

On a utilisé la souche microbienne *Aspergillus niger* ATCC 6275, et un milieu de culture liquide bouillon Sabouraud additionné de monopalmitate de sorbitane polyoxyéthyléné (20 OE) (Tween 40 de chez Croda) et de Phytagel© BioReagent, à double concentration.

On utilise une microplaque 96 puits à une température d'incubation de 32,5 °C.

La durée d'incubation de la microplaque pour les deux souches précitées est :

- de 18 à 24h en aérobiose pour *Candida albicans* et *Staphylococcus aureus*
- de 24 à 48h en aérobiose pour *Aspergillus niger ;*

**Essais**

Pour chaque composé :

A = composé 4-(3-éthoxy-4-hydroxyphényl)butan-2-one

B = composé additionnel.

On a préparé une solution mère à 10 % (poids/volume) en mélangeant 1 g de composé dans 9 ml de solution aqueuse d'agar à 1‰. Des dilutions successives ont été effectuées avec la solution d'agar à 1‰.

**Essais des composés A et B seul**

50 µL de chacune des solutions filles obtenues contenant le composé A ou B est ajouté dans les puits de la microplaque. On y ajoute également 100 µL de bouillon nutritif liquide de Sabouraud ensemencé à double concentration par la souche *Candida albicans* et 50 µL de solution aqueuse d'agar à 1‰.

**Essais des composés A et B en mélange**

50 µL de chacune des solutions filles obtenues contenant le composé A et 50 µL de chacune des solutions filles obtenues contenant le composé B sont ajoutées dans les puits de la microplaque. On y ajoute également 100 µL de bouillon nutritif liquide de Sabouraud ensemencé à double concentration par la souche *Candida albicans.*

**Témoin de croissance microbienne**

On a également réalisé un témoin positif de croissance microbienne. Le témoin positif de croissance microbienne correspond au mélange de 100 µL d'une solution aqueuse d'agar 1‰ avec 100 µL de bouillon nutritif liquide de Sabouraud ensemencé à double concentration par la souche *Candida albicans* en l'absence des composés A et B.

**Témoin d'absorbance des composés A et B seul**

On a réalisé parallèlement un témoin d'absorbance des composés A et B seul. Ce témoin correspond à 100 µL de bouillon nutritif liquide de Sabouraud stérile à double concentration + 100 µL du composé A ou B à double concentration.

Dans les trois cas (témoin absorbance, témoin de croissance et essai) le volume final présent dans chacun des puits de la microplaque est de 200 µL.

Dans les deux cas (essai et témoin), l'inoculum représente la concentration de la souche *Candida albicans* présente dans le volume final des puits (200 µL) et est compris entre 2 et $6.10^5$ UFC/mL en *Candida albicans.*

La concentration minimale inhibitrice (CMI) de chaque composé A et B seul et en association a été déterminée de façon connue à l'aide des mesures de densité optique à la longueur d'onde de 620 nm.

Le test tel que décrit ci-dessus (essais, témoins absorbance et témoin de croissance) a de nouveau été réalisé pour tester l'association A + B sur la souche *Staphylococcus aureus* et sur la souche *Aspergillus niger* le cas échéant.

On a obtenu les résultats suivants avec B1 = composé zinc pyrithione (solution à 48% en matière première dans l'eau en mélange avec 2 % de polynaphtalenesulphonate de sodium et 0.28% de gomme de cellulose*).

*Il est considéré que les additifs présents à si faible concentration n'ont pas d'influence sur les résultats de CMI obtenus.

***Staphyloccocus aureus***

| **Concentrations testées (en % poids)** | 0 A | 0,0625 A | 0,125 A | 0,25 A | **0,5 A** |
|---|---|---|---|---|---|
| 0 de B1 | | 87 | 77 | 78 | 4 |
| 0,00006 B1 | 34 | 42 | 51 | 40 | 1 |
| 0,00012 B1 | 26 | **11**<br>**(FIC 0.625)** | **7**<br>**(FIC 0.75)** | **1**<br>**(FIC 1)** | 0 |
| **0,00024 B1** | 0 | 0 | 1 | 0 | 0 |

| **CMI de A seul en %** | **CMI de B1 seul en %** | **CMI de chaque composé en mélange** | | **FIC Index** | **Ratio A/B1** |
|---|---|---|---|---|---|
| | | A % | B1 % | | |
| 0,5 | 0,00024 | 0,0625 | 0,00012 | 0,625 | 520 |

Les résultats obtenus montrent une synergie de l'activité inhibitrice pour les mélanges :

i) 0,0625 % de A et 0,00012 % de B1 soit ratio A/B1 = 520

ii) 0,125 % de A et 0,00012 % de B1 soit ratio A/B1 = 1041

iii) 0,25 % de A et 0,00012 % de B1 soit ratio A/B1 = 2083

***Candida albicans***

| **Concentrations testées (en % poids)** | 0 A | 0,025 A | 0,05 A | 0,1 A | **0,2 A** |
|---|---|---|---|---|---|
| 0 B1 | | 84 | 71 | 48 | 10 |
| 0,000006 B1 | 80 | 83 | 60 | 47 | 14 |
| 0,000012 B1 | 42 | 30 | 28 | 25 | 5 |
| 0,000024 B1 | 34 | **15 (FIC 0.625)** | **13 (FIC 0.75)** | **14 (FIC 1)** | 5 |
| **0,000048 B1** | 18 | 9 | 6 | 12 | 4 |

| CMI de A seul en % | CMI de B1 seul en % | CMI de chaque composé en mélange | | FIC Index | Ratio A/B1 |
|---|---|---|---|---|---|
| | | A % | B1 % | | |
| 0,2 | 0,000048 | 0,025 | 0,000024 | 1 | 1041 |

Les résultats obtenus montrent une synergie de l'activité inhibitrice pour les mélanges :

i) 0,025 % de A et 0,000024 % de B1 soit ratio A/B1 = 1041

ii) 0,05 % de A et 0,000024 % de B1 soit ratio A/B1 = 2083

iii) 0,1 % de A et 0,000024 % de B1 soit ratio A/B1 = 4166

**Exemple 2 : détermination de la synergie d'activité antimicrobienne en CMI sur la souche microbienne *Candida albicans***

La démonstration d'un effet de synergie d'activité antimicrobienne avec un mélange de 4-(3-éthoxy-4-hydroxyphényl)butan-2-one (appelée substance A) et de piroctone olamine (appelée substance B2) est réalisé selon le protocole décrit dans l'exemple 1.

On a obtenu les résultats suivants :

***Candida albicans***

| concentrations testées (en % poids) | 0 A | 0,025 A | 0,05 A | **0,1 A** |
|---|---|---|---|---|
| 0 B2 | | 52 | 31 | 18 |
| 0,00125 B2 | 35 | **21** | **14** | 7 |
| **0,0025 B2** | 0 | 0 | 1 | 0 |

| CMI A % | CMI B2 | CMI de composé A % | chaque en mélange B2 % | FIC Index | Ratio A/B2 |
|---|---|---|---|---|---|
| 0,1 | 0,005 | 0,025 | 0,00125 | 0,75 | 20 |

Les résultats obtenus montrent une synergie de l'activité inhibitrice pour les mélanges :

i) 0,025 % de A et 0,00125 % de B2 soit ratio A/B2 = 20

ii) 0,05 % de A et 0,00125 % de B2 soit ratio A/B2 = 40

**Exemple 3** : **Détermination de la synergie d'activité antimicrobienne en CMI**

La démonstration d'un effet de synergie d'activité antimicrobienne avec un mélange de 4-(3-éthoxy-4-hydroxyphényl)butan-2-one (appelée substance A) et du diazolidinylurée (appelée substance B3) est réalisée selon le protocole décrit dans l'exemple 1.

On a obtenu les résultats suivants :

***Candida Albicans***

| **Concentrations testées (en % poids)** | 0 A | 0,025 A | 0,05 A | 0,1 A | **0,2 A** |
|---|---|---|---|---|---|
| 0 B3 | | 71 | 65 | 44 | 4 |
| 0,0625 B3 | 55 | 26 | **17 (FIC 0,75)** | **7 (FIC 1)** | 1 |
| **0,125 B3** | 10 | 5 | 4 | 1 | 0 |

| CMI de A seul en % | CMI de B3 seul en % | CMI de chaque composé en mélange | | FIC Index | Ratio A/B3 |
|---|---|---|---|---|---|
| | | A% | B3 % | | |
| 0,2 | 0,125 | 0,05 | 0,0625 | 0,75 | 0,8 |

Les résultats obtenus montrent une synergie de l'activité inhibitrice pour les mélanges :

i) 0,05 % de A et 0,0625 % de B3 soit ratio A/B3 = 0,8

ii) 0,1 % de A et 0,0625 % de B3 soit ratio A/B3 = 1,6.

**Exemple 4 : Détermination de la synergie d'activité antimicrobienne en CMI**

La démonstration d'un effet de synergie d'activité antimicrobienne avec un mélange de 4-(3-éthoxy-4-hydroxyphényl)butan-2-one (appelée substance A) et du hexamidine diisethionate (appelée substance B4) est réalisée selon le protocole décrit dans l'exemple 1.

On a obtenu les résultats suivants :

***Candida Albicans***

| **Concentrations testées (en % poids)** | 0 A | 0,025 A | 0,05 A | 0,1 A | **0,2 A** |
|---|---|---|---|---|---|
| 0 B4 | | 83 | 71 | 46 | 6 |
| 0,00125 B4 | 78 | **4 (FIC 0,375)** | **1 (FIC 0,5)** | **0 (FIC 0,75)** | 1 |
| 0,0025 B4 | 59 | **0 (FIC 0,675 )** | **0 (FIC 0,75)** | **0 (FIC 1)** | 0 |
| **0,005 B4** | 3 | 0 | 0 | 0 | 0 |

| **CMI de A seul en %** | **CMI de B4 seul en %** | **CMI de chaque composé en mélange** | | **FIC Index** | **Ratio A/B4** |
|---|---|---|---|---|---|
| | | A % | B4 % | | |
| 0,2 | 0,005 | 0,025 | 0,00125 | 0,375 | 20 |

Les résultats obtenus montrent une synergie de l'activité inhibitrice pour les mélanges :

i) 0,025 % de A et 0,00125 % de B4 soit ratio A/B4 = 20

ii) 0,05 % de A et 0,00125 % de B4 soit ratio A/B4 = 40

iii) 0,1 % de A et 0,00125 % de B4 soit ratio A/B4 = 80

iv) 0,025 % de A et 0,0025 % de B4 soit ratio A/B4 = 10

v) 0,05 % de A et 0,0025 % de B4 soit ratio A/B4 = 20

vi) 0,1 % de A et 0,0025 % de B4 soit ratio A/B4 = 40.

**Exemple 5 : Détermination de la synergie d'activité antimicrobienne en CMI**

La démonstration d'un effet de synergie d'activité antimicrobienne avec un mélange de 4-(3-éthoxy-4-hydroxyphényl)butan-2-one (appelée substance A) et de l'imidazolidinylurée (appelée substance B5) est réalisée selon le protocole de l'exemple 1.

On a obtenu les résultats suivants :

***Aspergillus niger***

| **Concentrations testées (en % poids)** | 0 A | 0,0625 A | 0,125 A | **0,25 A** |
|---|---|---|---|---|
| 0 B5 | | 85 | 43 | 4 |
| 0,0625 B5 | 98 | 94 | 44 | 10 |
| 0,125 B5 | 97 | 92 | 40 | 5 |
| 0,25 B5 | 92 | 68 | **16 (FIC 0,75)** | 3 |
| 0,5 B5 | 38 | 24 | **5 (FIC 1)** | 1 |
| **1 B5** | 2 | 1 | 1 | 1 |

| **CMI de A seul en %** | **CMI de B5 seul en %** | **CMI de chaque composé en mélange** | | **FIC Index** | **Ratio A/B5** |
|---|---|---|---|---|---|
| | | **A %** | **B5 %** | | |
| 0,25 | 1 | 0,125 | 0,25 | 0,75 | 0,5 |

Les résultats obtenus montrent une synergie de l'activité inhibitrice pour le mélange :

i) 0,125 % de A et 0,25 % de B5 soit ratio A/B5 = 0,5
ii) 0,125 % de A et 0,5 % de B5 soit ratio A/B5 = 0,25

***Candida Albicans***

| **Concentrations testées (en % poids)** | 0 A | 0,025 A | 0,05 A | **0,1 A** |
|---|---|---|---|---|
| 0 B5 | | 56 | 39 | 17 |
| 0,0625 B5 | 43 | 38 | 26 | 15 |
| 0,125 B5 | 23 | **20 (FIC 0,75)** | **16 (FIC 1)** | 9 |
| **0,25 B5** | 17 | 14 | 12 | 6 |

| **CMI de A seul en %** | **CMI de B5 seul en %** | **CMI de chaque composé en mélange** | | **FIC Index** | **Ratio A/B5** |
|---|---|---|---|---|---|
| | | **A %** | **B5 %** | | |
| 0,1 | 0,25 | 0,025 | 0,125 | 0,75 | 0,2 |

Les résultats obtenus montrent une synergie de l'activité inhibitrice pour les mélanges :

i) 0,025 % de A et 0,125 % de B5 soit ratio A/B5 = 0.2

ii) 0,05 % de A et 0,125 % de B5 soit ratio A/B5 = 0.4

**Revendications**

1. Mélange antimicrobien comprenant de la 4-(3-éthoxy-4-hydroxyphényl)butan-2-one et un composé additionnel choisi parmi :

   i) un sel de métal monovalent ou divalent de la pyrithione ;
   ii) la piroctone olamine ;
   iii) le diazolidinylurée ;
   iv) un composé hexamidine choisi parmi le diiséthionate d'hexamidine, l'hexamidine diparabène et l'hexamidine parabène ;
   v) l'imidazolidinylurée.

2. Mélange selon la revendication précédente, **caractérisé en ce que** le composé additionnel est un sel de métal monovalent ou divalent de la pyrithione.

3. Mélange selon la revendication précédente, **caractérisé en ce que** le sel de métal monovalent ou divalent de la pyrithione est choisi parmi les sels de sodium, calcium, de magnésium, de baryum, de strontium, de zinc, de cadmium, d'étain et de zirconium.

4. Mélange selon l'une des revendications 2 ou 3, **caractérisé en ce que** le sel de métal monovalent ou divalent de la pyrithione est le sel de zinc et le sel de sodium.

5. Mélange selon l'une des revendications 2 à 4, **caractérisé en ce que** le sel de métal monovalent ou divalent de la pyrithione est le sel de zinc.

6. Mélange selon l'une des revendications 2 à 5, **caractérisé en ce qu'**il comprend du 4-(3-éthoxy-4-hydroxyphényl)butan-2-one et du sel de métal monovalent ou divalent de la pyrithione en quantités telles que le rapport pondéral 4-(3-éthoxy-4-hydroxyphényl)butan-2-one / sel de pyrithione va de 400 à 4500, et plus préférentiellement va de 500 à 4300.

7. Mélange antimicrobien selon les revendications 2 à 5, **caractérisé en ce qu'**il a un ratio pondéral 4-(3-éthoxy-4-hydroxyphényl)butan-2-one / sel de pyrithione allant de 400 à 2500, de préférence allant de 500 à 2200, de préférence allant de 1000 à 2200, et plus préférentiellement allant de 1800 à 2200.

8. Mélange antimicrobien selon les revendications 2 à 5, **caractérisé en ce qu'**il a un ratio pondéral 4-(3-éthoxy-4-hydroxyphényl)butan-2-one / sel de pyrithione allant de 800 à 4500, de préférence allant de 900 à 4300, et plus préférentiellement allant de 950 à 4200.

9. Mélange antimicrobien selon la revendication 1, **caractérisé en ce que** le composé additionnel est la piroctone olamine.

10. Mélange antimicrobien selon la revendication précédente, **caractérisé en ce qu'**il comprend du 4-(3-éthoxy-4-hydroxyphényl)butan-2-one et de la piroctone olamine en quantités telles que le rapport pondéral 4-(3-éthoxy-4-hydroxyphényl)butan-2-one / piroctone olamine va de 10 à 60, de préférence va de 15 à 50, et préférentiellement va de 18 à 45.

11. Mélange antimicrobien selon la revendication 1, **caractérisé en ce que** le composé additionnel est le diazolidinylurée.

12. Mélange selon la revendication précédente, **caractérisé en ce qu'**il comprend du 4-(3-éthoxy-4-hydroxyphényl)butan-2-one et du diazolidinylurée en quantités telles que le rapport pondéral 4-(3-éthoxy-4-hydroxyphényl)butan-2-one / diazolidinylurée va de 0,5 à 2,5, de préférence va de 0,6 à 2,3, de préférence va de 0,7 à 2, préférentiellement va de 0,7 à 1,8, et plus préférentiellement va de 1,2 à 1,8.

13. Mélange antimicrobien selon la revendication 1, **caractérisé en ce que** le composé additionnel est un composé hexamidine choisi parmi l'hexamidine diisethionate, l'hexamidine diparabène ou l'hexamidine parabène.

14. Mélange selon la revendication précédente **caractérisé en ce que** le composé hexamidine est l'hexamidine diisethionate.

15. Mélange selon l'une des revendications 13 ou 14, **caractérisé en ce qu'**il comprend la 4-(3-éthoxy-4-hydroxyphényl)butan-2-one et le composé hexamidine en quantités telles que le rapport pondéral 4-(3-éthoxy-4-hydroxyphényl)butan-2-one / composé hexamidine va de 5 à 85, de préférence va de 7 à 82, et préférentiellement va de 15 à 60.

16. Mélange antimicrobien selon la revendication 1, **caractérisé en ce que** le composé additionnel est l'imidazolidinylurée.

17. Mélange selon la revendication précédente, **caractérisé en ce qu'**il comprend du 4-(3-éthoxy-4-hydroxyphényl)butan-2-one et de l'imidazolidinylurée en quantités telles que le rapport pondéral 4-(3-éthoxy-4-hydroxyphényl)butan-2-one / imidazolidinylurée va de 0,1 à 0,8, et plus préférentiellement va de 0,15 à 0,45.

18. Mélange antimicrobien selon la revendication 16, **caractérisé en ce qu'**il a un ratio pondéral 4-(3-éthoxy-4-hydroxyphényl)butan-2-one / imidazolidinylurée allant de 0,2 à 0,8, et plus préférentiellement allant de 0,4 à 0,6.

19. Mélange antimicrobien selon la revendication 16, **caractérisé en ce qu'**il a un ratio pondéral 4-(3-éthoxy-4-hydroxyphényl)butan-2-one / imidazolidinylurée allant de 0,1 à 0,6, et plus préférentiellement allant de 0,15 à 0,45.

20. Composition comprenant, dans un milieu physiologiquement acceptable, un mélange antimicrobien selon l'une des revendications 1 à 19.

21. Composition selon la revendication précédente, **caractérisée en ce qu'**elle comprend au moins un ingrédient additionnel choisi parmi l'eau, les huiles, les polyols ayant de 2 à 10 atomes de carbone, les gélifiants, les tensioactifs, les polymères filmogènes, les matières colorantes, les parfums, les charges, les filtres UV, les extraits végétaux, les actifs cosmétiques et dermatologiques, et les sels.

**22.** Composition selon l'une des revendications 20 ou 21, **caractérisée en ce que** la 4-(3-éthoxy-4-hydroxyphényl)butan-2-one est présente en une teneur allant de 0,01 % à 5 % en poids, par rapport au poids total de la composition, de préférence allant de 0,01 % à 3 % en poids, préférentiellement allant de 0,01 à 2, 5 % en poids, et plus préférentiellement allant de 0,01 à 2 % en poids.

**23.** Procédé de traitement cosmétique non thérapeutique de soin et/ou de maquillage et/ou de nettoyage des matières kératiniques comprenant l'application sur lesdites matières kératiniques d'une composition selon l'une quelconque des revendications 20 à 22.

**24.** Procédé de conservation d'une composition comprenant un milieu physiologiquement acceptable, en particulier une composition cosmétique ou dermatologique, **caractérisé en ce qu'**il consiste à incorporer à ladite composition un mélange antimicrobien tel que défini à l'une des revendications 1 à 19.

**25.** Utilisation d'un mélange antimicrobien tel que défini à l'une des revendications 1 à 19 pour la conservation d'une composition comprenant un milieu physiologiquement acceptable.

**Patentansprüche**

**1.** Antimikrobielle Mischung, umfassend 4-(3-Ethoxy-4-hydroxyphenyl)butan-2-on und eine zusätzliche Verbindung, die aus

i) einem einwertigen oder zweiwertigen Metallsalz von Pyrithion,
ii) Piroctonolamin,
iii) Diazolidinylharnstoff,
iv) einer Hexamidin-Verbindung, die aus Hexamidindiisethionat, Hexamidindiparaben und Hexamidinparaben ausgewählt ist,
v) Imidazolidinylharnstoff

ausgewählt ist.

**2.** Mischung nach dem vorhergehenden Anspruch, **dadurch gekennzeichnet, dass** es sich bei der zusätzlichen Verbindung um ein einwertiges oder zweiwertiges Metallsalz von Pyrithion handelt.

**3.** Mischung nach dem vorhergehenden Anspruch, **dadurch gekennzeichnet, dass** das einwertige oder zweiwertige Metallsalz von Pyrithion aus Natrium-, Calcium-, Magnesium-, Barium-, Strontium-, Zink-, Cadmium-, Zinn- und Zirconiumsalzen ausgewählt ist.

**4.** Mischung nach einem der Ansprüche 2 oder 3, **dadurch gekennzeichnet, dass** es sich bei dem einwertigen oder zweiwertigen Metallsalz von Pyrithion um das Zinksalz und das Natriumsalz handelt.

**5.** Mischung nach einem der Ansprüche 2 bis 4, **dadurch gekennzeichnet, dass** es sich bei dem einwertigen oder zweiwertigen Metallsalz von Pyrithion um das Zinksalz handelt.

**6.** Mischung nach einem der Ansprüche 2 bis 5, **dadurch gekennzeichnet, dass** sie 4-(3-Ethoxy-4-hydroxyphenyl)butan-2-on und das einwertige oder zweiwertige Metallsalz von Pyrithion in solchen Mengen umfasst, dass das Gewichtsverhältnis von 4-(3-Ethoxy-4-hydroxyphenyl)butan-2-on zu Pyrithionsalz im Bereich von 400 bis 4500 und weiter bevorzugt im Bereich von 500 bis 4300 liegt.

**7.** Antimikrobielle Mischung nach den Ansprüchen 2 bis 5, **dadurch gekennzeichnet, dass** sie ein Gewichtsverhältnis von 4-(3-Ethoxy-4-hydroxyphenyl)butan-2-on zu Pyrithionsalz im Bereich von 400 bis 2500, vorzugsweise im Bereich von 500 bis 2200, vorzugsweise im Bereich von 1000 bis 2200 und weiter bevorzugt im Bereich von 1800 bis 2200 aufweist.

**8.** Antimikrobielle Mischung nach den Ansprüchen 2 bis 5, **dadurch gekennzeichnet, dass** sie ein Gewichtsverhältnis von 4-(3-Ethoxy-4-hydroxyphenyl)butan-2-on zu Pyrithionsalz im Bereich von 800 bis 4500, vorzugsweise im Bereich von 900 bis 4300 und weiter bevorzugt im Bereich von 950 bis 4200 aufweist.

9. Antimikrobielle Mischung nach Anspruch 1, **dadurch gekennzeichnet, dass** es sich bei der zusätzlichen Verbindung um Piroctonolamin handelt.

10. Antimikrobielle Mischung nach dem vorhergehenden Anspruch, **dadurch gekennzeichnet, dass** sie 4-(3-Ethoxy-4-hydroxyphenyl)butan-2-on und Piroctonolamin in solchen Mengen umfasst, dass das Gewichtsverhältnis von 4-(3-Ethoxy-4-hydroxyphenyl)butan-2-on zu Piroctonolamin im Bereich von 10 bis 60, vorzugsweise im Bereich von 15 bis 50 und bevorzugt im Bereich von 18 bis 45 liegt.

11. Antimikrobielle Mischung nach Anspruch 1, **dadurch gekennzeichnet, dass** es sich bei der zusätzlichen Verbindung um Diazolidinylharnstoff handelt.

12. Mischung nach dem vorhergehenden Anspruch, **dadurch gekennzeichnet, dass** sie 4-(3-Ethoxy-4-hydroxyphenyl)butan-2-on und Diazolidinylharnstoff in solchen Mengen umfasst, dass das Gewichtsverhältnis von 4-(3-Ethoxy-4-hydroxyphenyl)butan-2-on zu Diazolidinylharnstoff im Bereich von 0,5 bis 2,5, vorzugsweise im Bereich von 0,6 bis 2,3, vorzugsweise im Bereich von 0,7 bis 2, bevorzugt im Bereich von 0,7 bis 1,8 und weiter bevorzugt im Bereich von 1,2 bis 1,8 liegt.

13. Antimikrobielle Mischung nach Anspruch 1, **dadurch gekennzeichnet, dass** es sich bei der zusätzlichen Verbindung um eine Hexamidin-Verbindung, die aus Hexamidindiisethionat, Hexamidindiparaben und Hexamidinparaben ausgewählt ist, handelt.

14. Mischung nach dem vorhergehenden Anspruch, **dadurch gekennzeichnet, dass** es sich bei der Hexamidin-Verbindung um Hexamidindiisethionat handelt.

15. Mischung nach einem der Ansprüche 13 oder 14, **dadurch gekennzeichnet, dass** sie 4-(3-Ethoxy-4-hydroxyphenyl)butan-2-on und die Hexamidin-Verbindung in solchen Mengen umfasst, dass das Gewichtsverhältnis von 4-(3-Ethoxy-4-hydroxyphenyl)butan-2-on zu Hexamidin-Verbindung im Bereich von 5 bis 85, vorzugsweise von 7 bis 82 und bevorzugt von 15 bis 60 liegt.

16. Antimikrobielle Mischung nach Anspruch 1, **dadurch gekennzeichnet, dass** es sich bei der zusätzlichen Verbindung um Imidazolidinylharnstoff handelt.

17. Mischung nach dem vorhergehenden Anspruch, **dadurch gekennzeichnet, dass** sie 4-(3-Ethoxy-4-hydroxyphenyl)butan-2-on und Imidazolidinylharnstoff in solchen Mengen umfasst, dass das Gewichtsverhältnis von 4-(3-Ethoxy-4-hydroxyphenyl)butan-2-on zu Imidazolidinylharnstoff im Bereich von 0,1 bis 0,8 und weiter bevorzugt im Bereich von 0,15 bis 0,45 liegt.

18. Antimikrobielle Mischung nach Anspruch 16, **dadurch gekennzeichnet, dass** sie ein Gewichtsverhältnis von 4-(3-Ethoxy-4-hydroxyphenyl)butan-2-on zu Imidazolidinylharnstoff im Bereich von 0,2 bis 0,8 und weiter bevorzugt im Bereich von 0,4 bis 0,6 aufweist.

19. Antimikrobielle Mischung nach Anspruch 16, **dadurch gekennzeichnet, dass** sie ein Gewichtsverhältnis von 4-(3-Ethoxy-4-hydroxyphenyl)butan-2-on zu Imidazolidinylharnstoff im Bereich von 0,1 bis 0,6 und weiter bevorzugt im Bereich von 0,15 bis 0,45 aufweist.

20. Zusammensetzung, die in einem physiologisch unbedenklichen Medium eine antimikrobielle Mischung nach einem der Ansprüche 1 bis 19 umfasst.

21. Zusammensetzung nach dem vorhergehenden Anspruch, **dadurch gekennzeichnet, dass** sie mindestens einen zusätzlichen Inhaltsstoff umfasst, der aus Wasser, Ölen, Polyolen mit 2 bis 10 Kohlenstoffatomen, Geliermitteln, Tensiden, filmbildenden Polymeren, Farbmitteln, Duftstoffen, Füllstoffen, UV-Filtersubstanzen, Pflanzenextrakten, kosmetischen und dermatologischen Wirkstoffen und Salzen ausgewählt ist.

22. Zusammensetzung nach einem der Ansprüche 20 oder 21, **dadurch gekennzeichnet, dass** das 4-(3-Ethoxy-4-hydroxyphenyl)butan-2-on in einem Gehalt im Bereich von 0,01 bis 5 Gew.-%, bezogen auf das Gesamtgewicht der Zusammensetzung, vorzugsweise im Bereich von 0,01 bis 3 Gew.-%, bevorzugt im Bereich von 0,01 bis 2,5 Gew.-% und weiter bevorzugt im Bereich von 0,01 bis 2 Gew.-% vorliegt.

23. Nichttherapeutisches kosmetisches Behandlungsverfahren zum Pflegen und/oder Schminken und/oder Reinigen von Keratinmaterialien, bei dem man auf die Keratinmaterialien eine Zusammensetzung nach einem der Ansprüche 20 bis 22 aufbringt.

24. Verfahren zur Konservierung einer Zusammensetzung, die ein physiologisch unbedenkliches Medium umfasst, insbesondere einer kosmetischen oder dermatologischen Zusammensetzung, **dadurch gekennzeichnet, dass** es darin besteht, dass man in die Zusammensetzung eine antimikrobielle Mischung gemäß einem der Ansprüche 1 bis 19 einarbeitet.

25. Verwendung einer antimikrobiellen Mischung gemäß einem der Ansprüche 1 bis 19 zur Konservierung einer Zusammensetzung, die ein physiologisch unbedenkliches Medium umfasst.

## Claims

1. Antimicrobial mixture comprising 4-(3-ethoxy-4-hydroxyphenyl)butan-2-one and an additional compound selected from:

   i) a monovalent or divalent metal salt of pyrithione;
   ii) piroctone olamine;
   iii) diazolidinylurea;
   iv) a hexamidine compound selected from hexamidine diisethionate, hexamidine diparaben and hexamidine paraben;
   v) imidazolidinylurea.

2. Mixture according to the preceding claim, **characterized in that** the additional compound is a monovalent or divalent metal salt of pyrithione.

3. Mixture according to the preceding claim, **characterized in that** the monovalent or divalent metal salt of pyrithione is selected from the sodium, calcium, magnesium, barium, strontium, zinc, cadmium, tin and zirconium salts.

4. Mixture according to either of Claims 2 and 3, **characterized in that** the monovalent or divalent metal salt of pyrithione is the zinc salt and the sodium salt.

5. Mixture according to one of Claims 2 to 4, **characterized in that** the monovalent or divalent metal salt of pyrithione is the zinc salt.

6. Mixture according to one of Claims 2 to 5, **characterized in that** it comprises 4-(3-ethoxy-4-hydroxyphenyl)butan-2-one and monovalent or divalent metal salt of pyrithione in amounts such that the 4-(3-ethoxy-4-hydroxyphenyl)butan-2-one/pyrithione salt weight ratio is from 400 to 4500, and more preferentially from 500 to 4300.

7. Antimicrobial mixture according to Claims 2 to 5, **characterized in that** it has a 4-(3-ethoxy-4-hydroxyphenyl)butan-2-one/pyrithione salt weight ratio of from 400 to 2500, preferably of from 500 to 2200, preferably of from 1000 to 2200, and more preferentially of from 1800 to 2200.

8. Antimicrobial mixture according to Claims 2 to 5, **characterized in that** it has a 4-(3-ethoxy-4-hydroxyphenyl)butan-2-one/pyrithione salt weight ratio of from 800 to 4500, preferably of from 900 to 4300, and more preferentially of from 950 to 4200.

9. Antimicrobial mixture according to Claim 1, **characterized in that** the additional compound is piroctone olamine.

10. Antimicrobial mixture according to the preceding claim, **characterized in that** it comprises 4-(3-ethoxy-4-hydroxyphenyl)butan-2-one and piroctone olamine in amounts such that the 4-(3-ethoxy-4-hydroxyphenyl)butan-2-one/piroctone olamine weight ratio is from 10 to 60, preferably from 15 to 50, and preferentially from 18 to 45.

11. Antimicrobial mixture according to Claim 1, **characterized in that** the additional compound is diazolidinylurea.

12. Mixture according to the preceding claim, **characterized in that** it comprises 4-(3-ethoxy-4-hydroxyphenyl)butan-2-one and diazolidinylurea in amounts such that the 4-(3-ethoxy-4-hydroxyphenyl)butan-2-one/diazolidinylurea weight ratio is from 0.5 to 2.5, preferably from 0.6 to 2.3, preferably from 0.7 to 2, preferentially from 0.7 to 1.8, and more preferentially from 1.2 to 1.8.

**13.** Antimicrobial mixture according to Claim 1, **characterized in that** the additional compound is a hexamidine compound selected from hexamidine diisethionate, hexamidine diparaben or hexamidine paraben.

**14.** Mixture according to the preceding claim, **characterized in that** the hexamidine compound is hexamidine diisethionate.

**15.** Mixture according to either of Claims 13 and 14, **characterized in that** it comprises the 4-(3-ethoxy-4-hydroxyphenyl)butan-2-one and the hexamidine compound in amounts such that the 4-(3-ethoxy-4-hydroxyphenyl)butan-2-one/hexamidine compound weight ratio is from 5 to 85, preferably from 7 to 82, and preferentially from 15 to 60.

**16.** Antimicrobial mixture according to Claim 1, **characterized in that** the additional compound is imidazolidinylurea.

**17.** Mixture according to the preceding claim, **characterized in that** it comprises 4-(3-ethoxy-4-hydroxyphenyl)butan-2-one and imidazolidinylurea in amounts such that the 4-(3-ethoxy-4-hydroxyphenyl)butan-2-one/imidazolidinylurea weight ratio is from 0.1 to 0.8, and more preferentially from 0.15 to 0.45.

**18.** Antimicrobial mixture according to Claim 16, **characterized in that** it has a 4-(3-ethoxy-4-hydroxyphenyl)butan-2-one/imidazolidinylurea weight ratio of from 0.2 to 0.8, and more preferentially of from 0.4 to 0.6.

**19.** Antimicrobial mixture according to Claim 16, **characterized in that** it has a 4-(3-ethoxy-4-hydroxyphenyl)butan-2-one/imidazolidinylurea weight ratio of from 0.1 to 0.6, and more preferentially of from 0.15 to 0.45.

**20.** Composition comprising, in a physiologically acceptable medium, an antimicrobial mixture according to one of Claims 1 to 19.

**21.** Composition according to the preceding claim, **characterized in that** it comprises at least one additional ingredient selected from water, oils, polyols having from 2 to 10 carbon atoms, gelling agents, surfactants, film-forming polymers, colorants, perfumes, fillers, UV screening agents, plant extracts, active cosmetic and dermatological agents, and salts.

**22.** Composition according to either of Claims 20 and 21, **characterized in that** the 4-(3-ethoxy-4-hydroxyphenyl)butan-2-one is present in an amount of from 0.01% to 5% by weight, relative to the total weight of the composition, preferably of from 0.01% to 3% by weight, preferentially of from 0.01 to 2.5% by weight, and more preferentially of from 0.01 to 2% by weight.

**23.** Method for non-therapeutic, cosmetic care and/or make-up and/or cleansing treatment of keratinous substances, comprising the application to said keratinous substances of a composition according to any one of Claims 20 to 22.

**24.** Method for preserving a composition comprising a physiologically acceptable medium, in particular a cosmetic or dermatological composition, **characterized in that** it comprises incorporating into said composition an antimicrobial mixture as defined in one of Claims 1 to 19.

**25.** Use of an antimicrobial mixture as defined in one of Claims 1 to 19 for preserving a composition comprising a physiologically acceptable medium.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2011039445 A
- FR 2962333 A


### Littérature non-brevet citée dans la description


- *CHEMICAL ABSTRACTS,* 13463-41-7
- *CHEMICAL ABSTRACTS,* 68890-66-4
- *CHEMICAL ABSTRACTS,* 78491-02-8
- *CHEMICAL ABSTRACTS,* 659-40-5
- *CHEMICAL ABSTRACTS,* 39236-46-9
- **F.C. KULL ; P.C. EISMAN ; H.D. SYLWESTROWKA ; R.L. MAYER.** *Applied Microbiology,* 1961, vol. 9, 538-541